# EUROPEAN PATENT SPECIFICATION

(11) **EP 2 737 864 B1**
(45) Date of publication and mention of the grant of the patent: **12.04.2017**
(21) Application number: 14157391.5
(22) Date of filing: 10.12.2010
(51) Int. Cl.: A61B 17/70

(54) **Receiving part for receiving a rod for coupling the rod to a bone anchoring element and a bone anchoring device**
Aufnahmeteil für die Aufnahme einer Stange zur Kopplung der Stange in einem Knochenverankerungselement und Knochenverankerungsvorrichtung
Pièce de réception pour recevoir une tige pour coupler la tige sur un élément d'ancrage d'os et dispositif d'ancrage d'os

(43) Date of publication of application: 04.06.2014
(62) Divisional of application: 10194596.2
(73) Proprietor: Biedermann Technologies GmbH & Co. KG, 78166 Donaueschingen (DE)
(72) Inventor: Biedermann, Lutz, 78048 VS-Villingen (DE); Matthis, Wilfried, 79367 Weisweil (DE)
(74) Representative: Prüfer & Partner mbB Patentanwälte · Rechtsanwälte

(56) References cited:
- EP-A1- 2 204 129
- WO-A2-2004/089245
- WO-A2-2007/038350
- US-A1- 2001 047 173
- US-A1- 2002 032 443

## Description

The invention relates to a receiving part for receiving a rod for coupling the rod to a bone anchoring element and a bone anchoring device with such a receiving part. The receiving part includes a receiving part body with a rod receiving portion and a head receiving portion for receiving the head of the bone anchoring element and a locking ring for locking the head in the head receiving portion. The head can be clamped by compressing a plurality of flexible wall sections with the locking ring wherein the clamping force is generated in a wall section at a circumferentially distinct pressure area.

US 5,733,285 describes a polyaxial colletted locking mechanism for use with an orthopaedic apparatus including a screw having a curvate head and a coupling element. The coupling element has a tapered and colletted portion having an interior chamber in which the curvate head is initially polyaxially disposed. A locking collar is disposed around the tapered and colletted portion such that translation thereof in the direction of the expanding taper causes the interior volume to contract onto the curvate head and lock it therein.

WO 2007/038350 A2 discloses an apparatus for connecting a bone anchor to a support rod, the apparatus including a connector body and a cap. The connector body has a socket for insertion, angulation and removal of a bone anchor. A sleeve is provided which is configured to fit over the connector body for locking the bone anchor in the socket.

EP 2 204 129 A1 also discloses an apparatus for connecting a bone anchor to a support rod. The apparatus comprises a receiving part body with a head receiving portion having an open end and being flexible so as to allow introduction and clamping of the head. The head receiving portion is provided with an outer tapered surface. A locking ring embracing the head receiving portion has a tapered inner surface exerting a force onto the head receiving portion to lock the head therein.

US 2001/004713 A1 shows a device for connecting a longitudinal support to a bone anchor having a rounded head. The device has a lower portion with a chamber for receiving the rounded head. The lower portion has a substantially spherical exterior surface. A sleeve is slidable over the body for compressing the chamber for biasing the longitudinal support. A lower sleeve acts as a clamping ring and it is fitted with a conical inside wall and is slidable over the exterior surface of a lower portion to compress the chamber.

WO 2004/089245 A2 describes a bone anchor having a bottom ring. The bottom ring surrounds a lower portion of a housing provided to accept a rod and join it with the polyaxial bone anchor. The lower portion has multiple resiliently opposed fingers and further comprises a convex outer surface. An inner surface of the bottom ring lies on the outside of the outer diameter of the lower portion.

US 2002/0032443 A1 discloses a multi-axial screw assembly including a bone screw mounted within a receiver member. A pair of shape-memory alloy locking rings is provided to clamp the receiver member about the bone screw.

It is the object of the invention to provide an improved receiving part for receiving a rod for coupling the rod to a bone anchoring element and a bone anchoring device with such a receiving part which allows a save handling during surgery and a save fixation of the bone anchoring element and the rod.

The object is solved by a receiving part according to claim 1 and bone anchoring device according to claim 13. Further developments are given in the dependent claims.

The receiving part according to the invention is improved in view of the clamping and locking of the head. Exerting pressure by the locking ring at positions where the head receiving portion has slits does not contribute to an effective clamping of the head. Therefore, the bone anchoring device according to the present invention is designed such that the pressure force exerted by the locking ring onto the head receiving portion is concentrated onto distinct pressure areas separated from each other in circumferential direction. Hence, the clamping force can be precisely applied which improves the safety of the fixation.

Furthermore, the receiving part according to the invention can have a pre-locking function wherein the locking ring is latched with respect to the receiving part body in a condition in which the head is inserted but not yet locked so that the head is prevented from removal.

With the bone anchoring device, a modular system can be provided that allows to combine various anchoring elements with any suitable receiving part on demand depending on the actual clinical requirements. The reduces the costs of polyaxial screws, reduces the inventory and gives the surgeon a substantial choice of implants.

Further features and advantages of the invention will become apparent from the description of embodiments by means of the accompanying drawings.

In the drawings
- Fig. 1: shows a perspective exploded view of a first embodiment of the bone anchoring device.
- Fig. 2: shows a perspective view of the bone anchoring device of fig. 1 in an assembled state.
- Fig. 3a) to 3e): show a perspective view, a side view, a first cross-sectional view, a second cross-sectional view and a bottom view, respectively, of the receiving part body of the bone anchoring device according to the first embodiment.
- Fig. 4a) to 4d): show a perspective view, a side view, a bottom view and a cross-sectional view, respectively, of the locking ring of the bone anchoring device according to the first embodiment.
- Fig. 5a) to 5c): show cross-sectional views of steps of use of the bone anchoring device according to the first embodiment.
- Fig. 6: shows a perspective exploded view of a second example of the bone anchoring device.
- Fig. 7: shows a perspective view of the bone anchoring device of Fig. 6 in an assembled state.
- Fig. 8a) to 8d): show a perspective view, a side view, a cross-sectional view and a bottom view, respectively, of the receiving part body of the bone anchoring device of the second example.
- Fig. 9a) to 9d): show a perspective view, a bottom view, a side view and a cross-sectional view, respectively, of the locking ring of the bone anchoring device of the second example.
- Fig. 10a) to 10c): show cross-sectional views of steps of use of the bone anchoring device according to the second example.
- Fig: 1.1: shows a perspective exploded view of a third example of the bone anchoring device.
- Fig. 12: shows a perspective view of the bone anchoring device of Fig. 11 in an assembled state.
- Fig. 13a) to 13d): show a perspective view, a side view, a cross-sectional view and a bottom view, respectively, of the receiving part body of the bone anchoring device according to the third example.
- Fig. 14a) to 14d): show a perspective view, a bottom view, a side view and a cross-sectional view, respectively, of the locking ring of the bone anchoring device according to the third example.
- Fig. 15a) to 15c): show cross-sectional views of different steps of use of the bone anchoring device according to the third example.

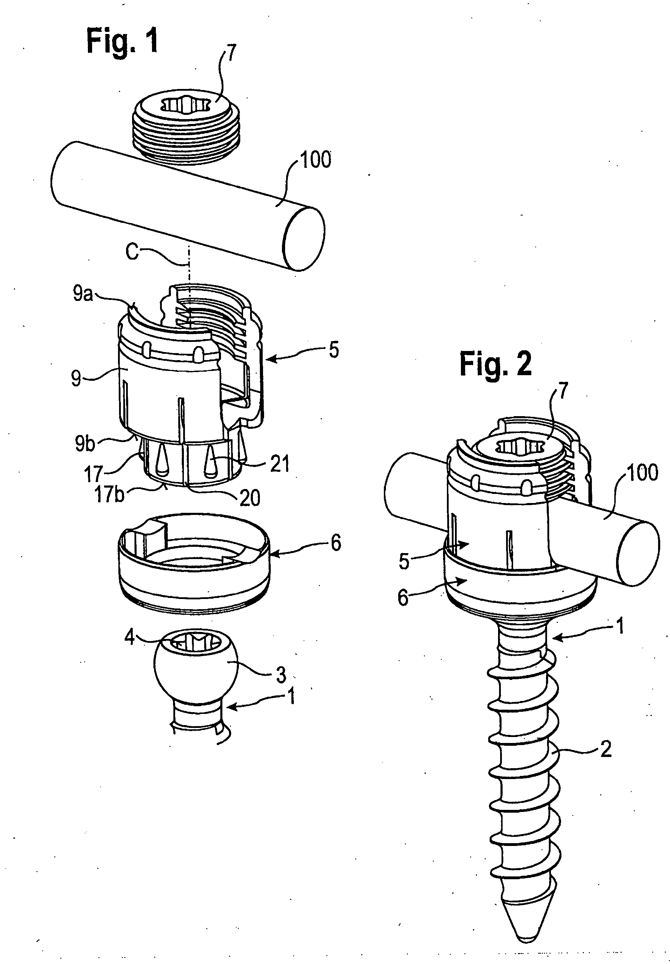

A first embodiment of a bone anchoring device illustrating the invention is shown in Figs. 1 to 5c). The remaining figures illustrate examples useful for understanding the invention. The bone anchoring device includes a bone anchoring element 1 in the form of a bone screw having a threaded shaft 2 and a head 3 with a spherically-shaped outer surface portion. The head 3 has a recess 4 for engagement with a driver. The bone anchoring device further includes a receiving part body 5 for receiving a rod 100 to connect the rod 100 to the bone anchoring element 1. For locking the head 3 in the receiving part body 5 a locking ring 6 is provided that engages the receiving part body 5. Furthermore, a fixation device in the form of an inner screw 7 is provided for fixing the rod 100 in the receiving part body 5.

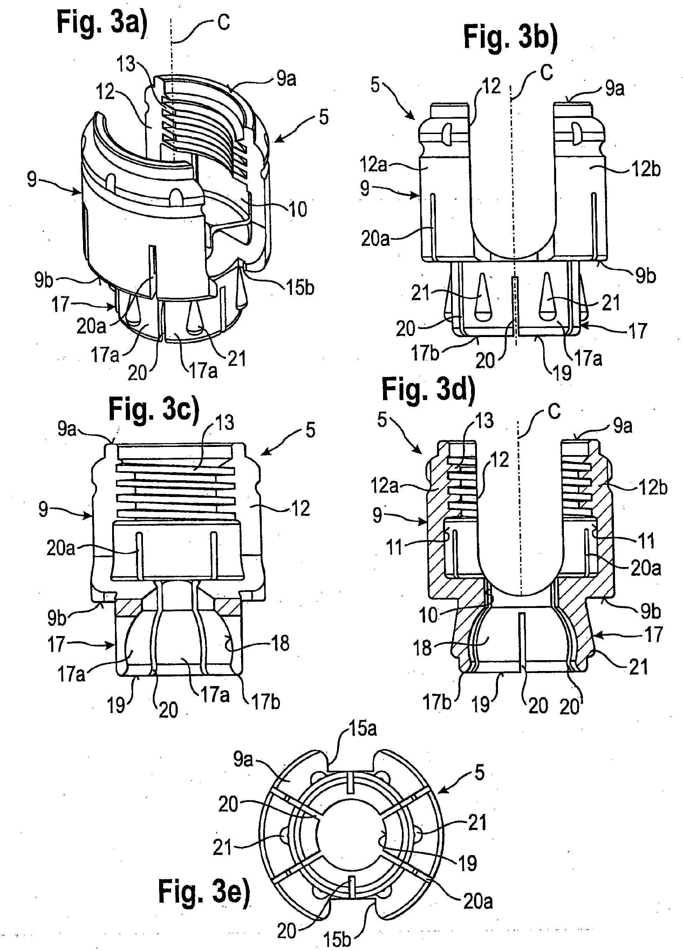

In Figs. 3a) to 3e), Fig. 3c) is a cross-sectional view wherein the section is being taken in a plane containing the rod axis and Fig. 3d) is a cross-sectional view wherein the section is taken perpendicular to the rod axis. As shown in particular in Figs. 1 to 3e), the receiving part body 5 comprises a rod receiving portion 9 which is substantially cylindrical and has a first end 9a and an opposite second end 9b and a central axis or cylinder axis C. A coaxial first bore 10 is provided at the second end 9b. The diameter of the first bore 10 is smaller than the diameter of the head 3 of the bone anchoring element. A coaxial second bore 11 extends from the first end 9a to a distance from the second end 9b. The diameter of the second 11 is greater than that of the first bore 10. A substantially U-shaped recess 12 extends from the first end 9a in the direction of the second end 9b in the rod receiving portion, the diameter of the recess 12 being slightly larger than the diameter of the rod 100 to allow placing the rod 100 in the recess and guiding it therein. By means of the recess 12 to free legs 12a, 12b are formed on which an internal thread 13 is provided. The internal thread 13 can be a metric thread, flat thread, a negative angle thread, a saw-tooth thread or any other thread form. If the fixation device is in form of the inner screw 7, a thread form such as a flat thread or a negative angle thread can be used which prevents splaying of the legs 12a, 12b when the inner screw 7 is tightened. The depth of the recess 12 is such that the rod 100 and the inner screw 7 can be inserted between the legs 12a, 12b. It shall be noted that the diameter of the coaxial bore 11 can vary along the central axis C.

As can be seen in particular in Figs. 3a) and 3e) cut-outs 15a,15b are provided in the rod receiving portion on either end of the channel formed by the recess 12.

At the side of the second end 9b the receiving part body 5 comprises a head receiving portion 17 providing an accommodation space for the head 3 of the bone anchoring element 1. The head receiving portion 17 is substantially cylindrically-shaped with an outer diameter which is smaller than the greatest outer diameter of the rod receiving portion 9 such that the head receiving portion 17 is recessed with respect to the rod receiving portion 9 as can be best seen, for example in Figs. 3b) and 3c). An internal hollow section 18 in the head receiving portion 17 forms a seat for the head of the bone anchoring element 1. The hollow section 18 is open via the opening 19 to a free end 17b of the head receiving portion. In the embodiment shown, the hollow section 18 is spherically shaped to accommodate the spherical head 3. However, the hollow section 18 generally may be adapted to any other shape of the head 3 or can be shaped otherwise so as to allow locking of the head 3 in the head receiving portion 17.

A plurality of slits 20 are provided in the head receiving portion 17. The slits 20 extend from the free end 17b to a distance from the second end 9b of the rod receiving portion. Generally, the slits 20 extend over a region which includes the largest inner diameter of the hollow section 18. The slits 20 render the head receiving portion 17 flexible so that it can be compressed to clamp and finally lock the head 3 in the internal hollow section 18 by friction. The head receiving portion 17 is configured to allow the insertion of the head 3 by expanding the head receiving portion and to clamp and finally lock the head 3 by compressing the head receiving portion. 17. Some of the slits 20 extend further into the rod receiving portion forming slits 20a as shown, for example, in Figs. 3a) and 3b). By means of this, the insertion of the head 3 can be facilitated further.

By the slits 20, 20a flexible wall sections 17a of the head receiving portion 17 are formed. On each of the flexible wall sections 17a a pressure area is formed in which the pressure generated by the cooperation with the locking ring 6 with the head receiving portion 17 is applied to the head 3. The pressure area is a distinct area seen in a circumferential direction within the flexible wall section 17a. In the first embodiment, the pressure area is formed by a tear drop-shaped projection 21 at the outer surface of each flexible wall section 17a. The tear drop-shaped projection 21 is arranged at the center of the flexible wall section 17a in an axial direction and in a circumferential direction. The tear drop-shaped projection 21 is oriented such that its height and width increases towards the open end 17b. By means of this, the outer diameter of the head receiving portion increases towards the free end 17b in the region of the tear drop-shaped projection as can be seen best in Fig. 3d). Hence, an outwardly tapered pressure area is provided at each of the flexible wall sections 17a.

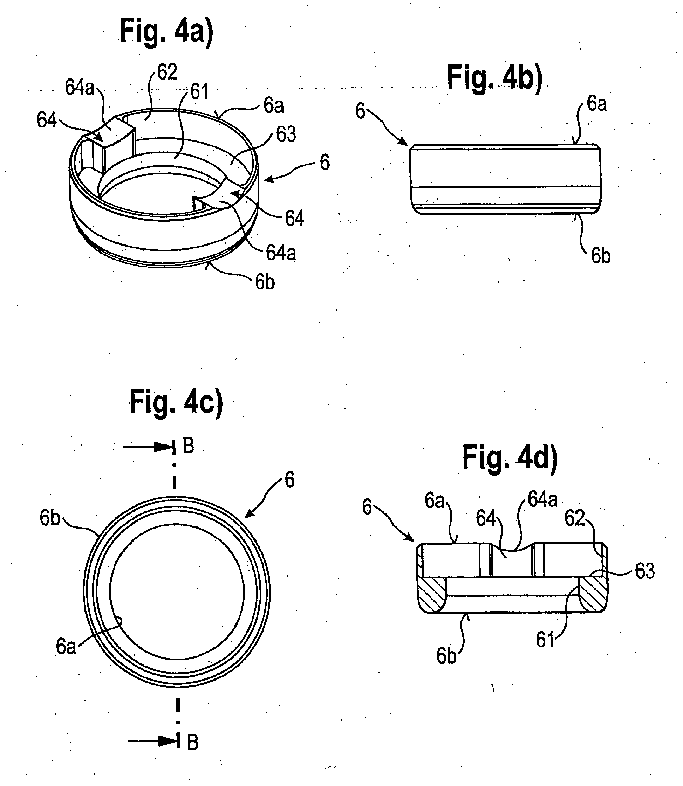

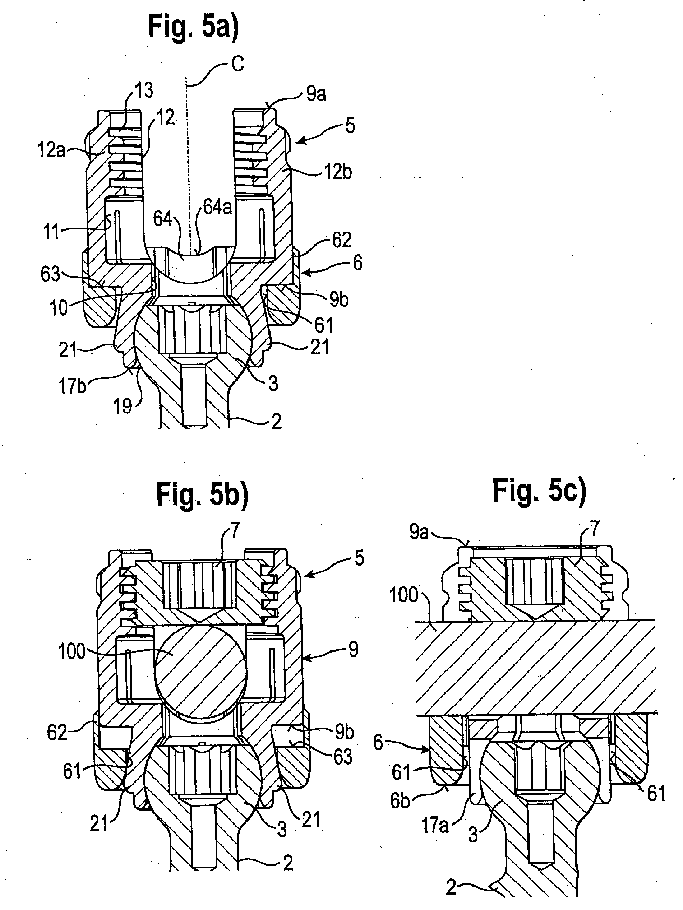

The locking ring 6 will now be described with reference to Figs. 4a) to 4d). Fig. 4a) shows a perspective view of the locking ring 6. The locking ring 6 is substantially cylindrical and has an upper end 6a and a lower end 6b. In the mounted state the upper end 6a is oriented in the direction of the first end 9a of the rod receiving portion and the lower end 6b is oriented towards the free end 17b of the head receiving portion. Adjacent the lower end 6b the locking ring 6 is substantially hollow cylindrically-shaped with an inner cylindrical surface 61, the diameter of which is slightly smaller than the outer diameter of the head receiving portion 17 in the region of the outermost area of the tear drop-shaped projection 21. By means of this, the head receiving portion 17 can be compressed to clamp and finally lock the head 3 when the locking ring 6 is around the head receiving portion 17. The lower end 6b of the locking ring may have a rounded edge as shown, for example in Fig. 4d) which shows a sectional view of the locking ring according line B-B in Fig. 4c). Adjacent the cylindrical surface 61 the locking ring 6 has a circular rim 62 with the upper end 6a. Between the circular rim 62 and the hollow cylindrical surface 61 an annular abutment surface 63 is formed. The height of the circular rim 62 is such that when the locking ring 6 is in its lowermost position the rim 62 bridges the gap between the abutment surface 63 and the second end 9b as shown, in particular in Fig. 5b). This may serve, for example, for preventing tissue growing into the gap between the abutment surface 63 and the second end 9b of the receiving part. The locking ring 6 further has two projections 64 as shown in particular in Fig. 4a) and Fig. 4d) which project from the abutment surface 63 towards the upper end 6a. The projections 64 are offset from each other by 180° and may have a concave upper surface 64a for facilitating receiving the rod 100.

All parts of the bone anchoring device are made of a bio-compatible material, for example of titanium, stainless steel or a bio-compatible alloy, such as Nitinol, or a bio-compatible plastic material such as, for example polyetheretherketone (PEEK). The parts can all be made of the same or of different materials.

The assembly and use of the bone anchoring device will now be explained with reference to Figs. 5a) to 5c). The locking ring 6 is mounted from the free end 17b of the head receiving portion by compressing the flexible wall sections 17a. Next, the locking ring is moved into a first position, which is the insertion position, shown in Fig. 5a). In this position, the annular abutment surface 63 abuts against the lower end 9b of the rod receiving portion 9. Between the inner cylindrical surface 61 and the outer surface of the head receiving portion 17 there is a gap which allows the expansion of the flexible wall section 17a to some extent. In this condition, the head 3 can be inserted from the free end 17b by expanding the hollow internal section 18. Once the head 3 is inserted to the hollow internal section 18, the locking ring 6 is prevented from falling out, since the outer diameter of the head receiving portion 17 in the area of the highest point of the tear drop-shaped projection 21 is larger than the inner diameter of the cylindrical surface 61. The projections 64 of the locking ring aligned with the U-shaped recess 12. As can be seen in Fig. 5a) in the first position, the projections 64 project above the bottom of the U-shaped recess 12.

In this condition, which can be achieved by preassembling the receiving part body 5, the locking ring 6 and the anchoring element 1, the anchoring element 1 is inserted into a bone part or a vertebra. The recess 4 of the head can be accessed with a driver through the first bore 10. In the condition shown in Fig. 5a) the receiving part is still pivotable relative to the head 3 of the bone anchoring element 1. Usually, at least two bone anchoring devices are used which are connected to the stabilization rod 100. After insertion of each of the bone anchoring devices, the receiving part bodies 5 are rotated and/or pivoted to be adjusted to receive the rod 100. Once the correct position of the rod with respect to the bone anchoring devices is achieved, the inner screw 7 is screwed between the legs 12a, 12b until it presses onto the rod 100. The rod 100 is thereby shifted towards the bottom of the U-shaped recess thereby engaging the upper surface 64a of the projections 64 and shifting down the locking ring 6.

When the locking ring 6 is shifted towards the free end 17b of the head receiving portion its cylindrical inner surface 62 engages the outwardly tapering surface of the tear drop-shaped projections 21, thereby creating an increasing pressure onto the flexible wall sections 17a. When the locking ring has been fully moved downwards the pressure exerted by the locking ring onto the flexible wall sections is such that the head 3 is finally locked in the hollow internal section 18.

As can be seen in Fig. 5c) which shows a cross-sectional view of the bone anchoring device with inserted rod and tightened inner screw 7 in a section containing the rod axis, the locking ring does not engage the flexible wall section 17a other than at the distinct pressure areas realized by the tear drop-shaped projections 21. On other areas, there is a gap between the parallel surfaces of the inner cylindrical surface 61 and the flexible wall sections 17a. Hence, the pressure is applied only at the distinct pressure areas which are evenly distributed in a circumferential direction. Pressure is not exerted at positions which do not contribute to clamping the head such as the slits 20. Therefore, the efficiency of applying the clamping force is increased.

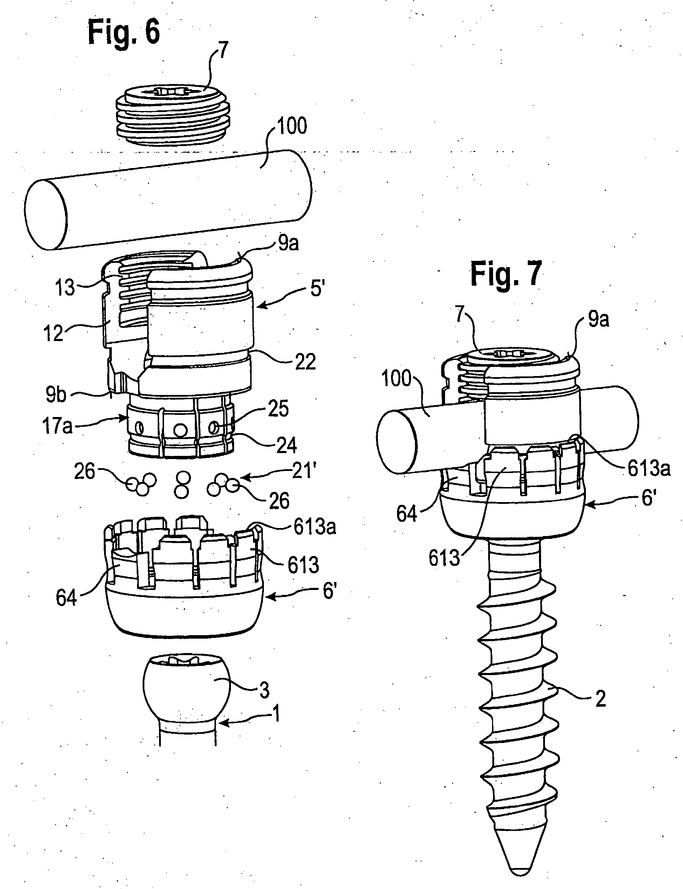

A second example of the bone anchoring device will now be described with reference to Figs. 6 to 10c). Parts and portions which are identical or similar to the parts and portions of the first embodiment are described by the same reference numerals and the description thereof will not be repeated. The bone anchoring device of the second example differs from the bone anchoring device according to the first embodiment essentially be the design of the pressure area and of the locking ring.

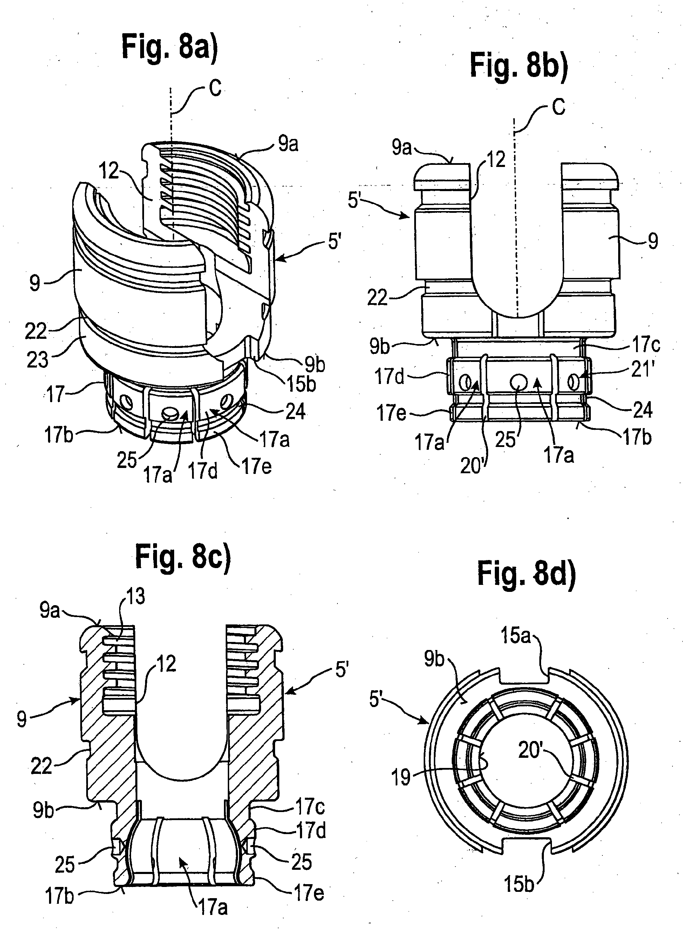

As can be seen in particular in Figs. 1 and 8a) to 8d) the receiving part body 5' has at its outer surface of the rod receiving portion 9 an engagement portion for the locking ring in the form of a circumferential groove 22 and a cylindrical portion 23 with slightly reduced diameter compared to the substantially cylindrical portion on the upper side of the groove 22. The groove 22 acts as a stop for the locking ring in a second position which is a pre-locking position described below.

The head receiving portion 17 comprises the pressure areas which are realized by a ball bearing 21' provided in each flexible wall section, respectively. The head receiving portion 17 has a first cylindrical section 17c with a first diameter adjacent the second end 9b of the rod receiving portion and a second cylindrical portion 17d with a second diameter greater than the first diameter which has the ball bearing 21'. A third portion 17e with a diameter that is essentially the same as that of the second portion 17c is provided at the free end 17b. Between the second portion 17d and the third portion 17e a groove 24 is provided that serves for engagement with a portion of the locking ring. It shall be noted that the grooves 22 and 24 may have an inclined lower edge which is inclined towards the free end 17b to facilitate disengagement of the locking ring when moving downwards. The second portion 17c is substantially at a position around the largest diameter of the hollow internal section 18 which corresponds to the largest outer diameter of the head 3 when it is inserted into the hollow interior section 18. The slits 20' preferably extend from the free end 17b only as far as the upper end of the second portion 17d, as for example, shown in Figs. 8b) and 8c).

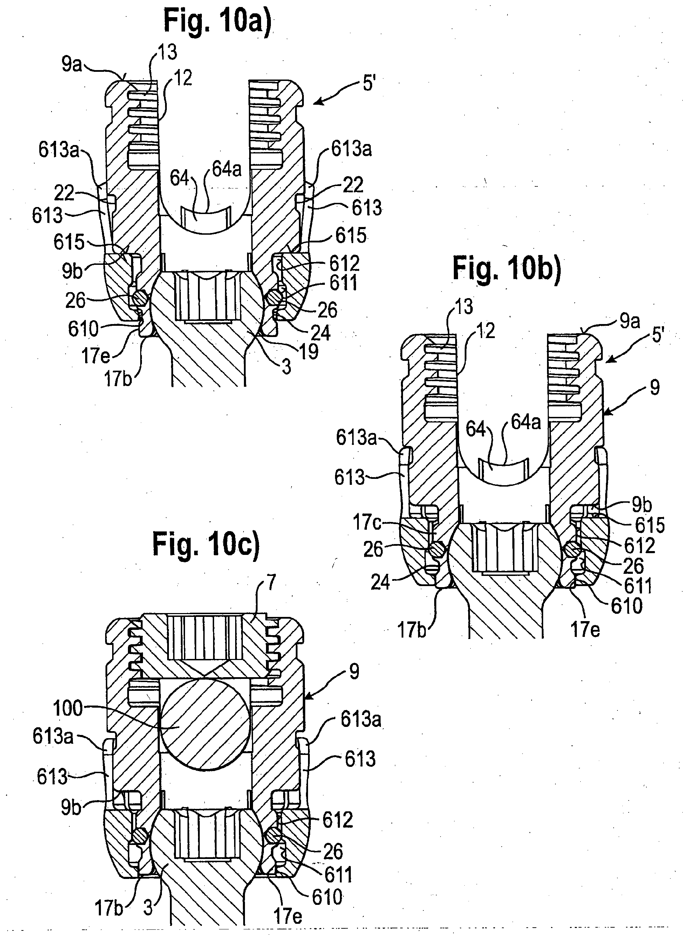

Each of the flexible wall sections 17a comprises a distinct pressure area in form of a ball bearing21' at its center in a circumferential direction. Each ball bearing 21' includes a recess 25 having a substantially circular cross section which is sized and shaped to accommodate a ball 26 therein. The ball 26 can rotate in the recess and a portion of the ball projects out of the second cylindrical portion 17d. As soon as the locking ring is mounted the balls cannot fall out. As best seen in Figs. 6 and 10a) to 10c) the receiving part body 5' comprises in a circumferential direction equidistantly spaced balls 26 that cooperate with the locking ring.

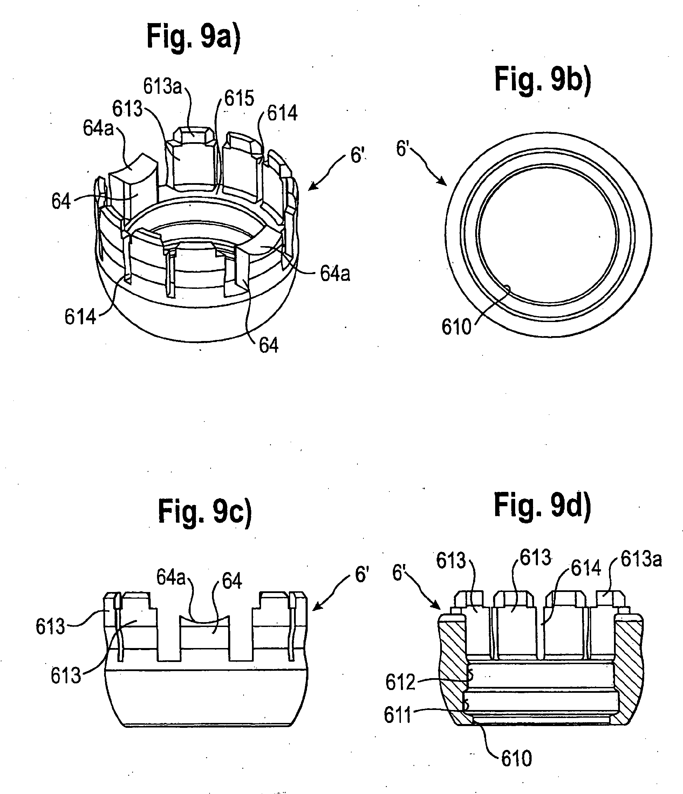

The locking ring will now be described with respect to Figs. 9a) to 9d). The locking ring 6' is substantially cylindrical and has the upper end 6a and the lower end 6b. Near the lower end 6b an inwardly projecting circular edge 610 is provided that cooperates with the third portion we adjacent the free end 17b of the head receiving portion 17. The inwardly projecting edge 610 provides a cylindrical inner surface. Furthermore, the inwardly projecting edge 610 is configured to engage the groove 24 of the head receiving portion 17.

Adjacent the inwardly projecting edge 610 the locking ring 6' comprises a hollow cylindrical portion 611 the height of which is such that it can receive the portions of the balls 26 protruding out of the recesses 25, as can be seen in particular in Fig. 10a). Hence, the inner diameter is larger than the inner diameter of the inwardly projecting edge 610. Adjacent the hollow cylindrical portion 611, there is a hollow cylindrical portion 612, the inner diameter of which is smaller than the inner diameter of the hollow cylindrical portion 611 and larger than the inner diameter of the inwardly projecting edge 610. The inner diameter of the hollow cylindrical portion 612 is such that, as can be seen in Fig. 10b) when the locking ring is around the head receiving portion 17 such that the hollow cylindrical portion 612 is arranged around the balls 26, the inner surface of the hollow cylindrical portion 612 can slide along the balls of the ball bearing.

The upper portion of the locking ring 6' adjacent the upper end 6a consists of upwardly extending wall portions 613 which are separated from each other by slits 614. The upwardly extending wall portions 613 are arranged at the outer circumference of an inner circumferential shoulder 615 of the locking ring and render the upper portion of the locking ring flexible. The number and the size of the slits and the thickness of the wall portions 613 are configured such that a desired flexibility is obtained. At their free ends the wall portions 613 comprise engagement sections 613 a which are shaped so as to engage the groove 22 provided on the outer surface of the rod receiving portion 9.

The locking ring 6' is sized in such a way with respect to the head receiving portion 17 that the head receiving portion 17 can expand within the locking ring 6' to allow the introduction of the head 3 when the locking rings in the first position as shown in Fig. 10a).

Two projections 64 which may have a concave upper surface portion 64a are provided at 180° offset from each other like in the locking ring 6 of the first embodiment. The curvature of the upper surface 64a may correspond to the curvature of the rod 100. The projections 64 have such a height that they project above the bottom of the U-shaped recess 12 and extend into the cut-outs 15a, 15b, when the locking ring 6' is in a position in which the head 3 is not yet locked as shown in Figs. 10a) and 10b). The height of the upwardly extending wall portions 613 is such that when the locking ring is in a first position shown in Fig. 10a) in which its inner circumferential shoulder abuts against the second end 9b, the upwardly extending wall portion 613 are with their engagement portion 613a above the circumferential groove 22. At the outside, the locking ring may be tapered towards its lower end 6b to reduce the outer dimension of the receiving part.

The function and use of the bone anchoring device will now be explained with reference to Figs. 10a) to 10c). As shown in Fig. 10a), a first position of the locking ring 6', which is the insertion position in which the locking ring 6' is latched with respect to the receiving part body 5', is defined in such a way that the inwardly projecting edge 610 engages the groove 24 at the outer surface of the head receiving portion 17. In this condition, the head 3 can be inserted through the opening 19 into the hollow internal section 18 of the head receiving portion 17. Since the inner diameter of the inwardly projecting edge 610 is larger than the outer diameter of the groove 24, an expansion of the head receiving portion 17 when the head 3 is introduced is possible. In the first position, the locking ring 6' is additionally held by a clamping force between the rod receiving portion 9 and the flexible wall portions 613 of the locking ring 6' which are slightly bent outwards as can be seen in particular in Fig. 10a).

When the locking ring is in the first position, the head receiving portion 17 is not compressed. In this position, the locking ring is prevented from moving upwards towards the first end 9a of the rod receiving portion, since it abuts with the shoulder 615 against the second end 9b of the head receiving portion and with the inwardly projecting edge 610 against the upper wall of the groove 24. This holds the locking ring 6' in place. The inclined lower edge of the groove 24 prevents an inadvertent downward movement of the locking ring 6' but allows a downward movement upon exertion of an additional downwardly directed force. In the first position, the head can freely pivot in the hollow internal section 18. The head receiving portion is not compressed and the balls 26 are not touched, since they project into the hollow cylindrical portion 611.

A second position, in which the locking ring 6' is latched with respect to the receiving part body 5' is shown in Fig. 10b). A second position is a pre-locking position in which the head 3 is prevented from removal from the hollow internal section 18 and optionally is held in a preliminary angular position by a slight friction force exerted by the flexible wall section 17a. In the second position, the locking ring 6' has been shifted towards the free end 17b of the head receiving portion until the engagement portion 613a of the upwardly extending wall portion 613 resiliently snap into the groove 22 provided at the rod receiving portion 9. The free upper edge of the engagement portions 613a abut against the upper wall of the groove 22, as shown in Fig. 10b), thereby preventing upward movement of the locking ring out of the pre-locking position. On the other hand, the inclined lower edge of the groove 22 prevents inadvertent downward movement of the locking towards the free end 17b but allows such downward movement upon exertion of an additional axial force.

To reach the second position from the first position the locking 6' has to be shifted downwards. While the locking ring is shifted downwards the hollow cylindrical portion 612 slides along the rotating balls 26, thereby exerting pressure onto the flexible wall section 17a at the position of the balls 26. Hence, a jamming of the two cylindrical surfaces 17d of the head receiving portion and the hollow cylindrical portion 612 of the locking ring is prevented while simultaneously pressure is exerted onto the balls 26 which project out from the surface 17d. The balls 26 define distinct pressure areas to exert the pressure onto the head 3 to clamp and finally lock the head 3.

In the pre-locked position, the bone anchoring element cannot be removed from the receiving part. Hence, an accidental or inadvertent removal of the head is not possible. However, an angulation of the screw element is still possible by overcoming the friction force, for example manually.

A third position, which is the locking position, is shown in Fig. 10c). The third position is defined as the position in which the head 3 is finally locked within the receiving portion 17. The inwardly projecting edge 610 compresses the third portion 17e adjacent the free end 17b of the head receiving portion 17. The combination of the pressure exerted via the pressure areas in form of the balls 26 and the pressure exerted via the inwardly projecting edge 610 firmly locks the head 3 within the receiving part body 5. The third position is reached by a downward movement of the locking ring upon the action of the rod 100 pressing the upper surface of the projections 64. The rod is pressed downward when tightening the fixation screw 7.

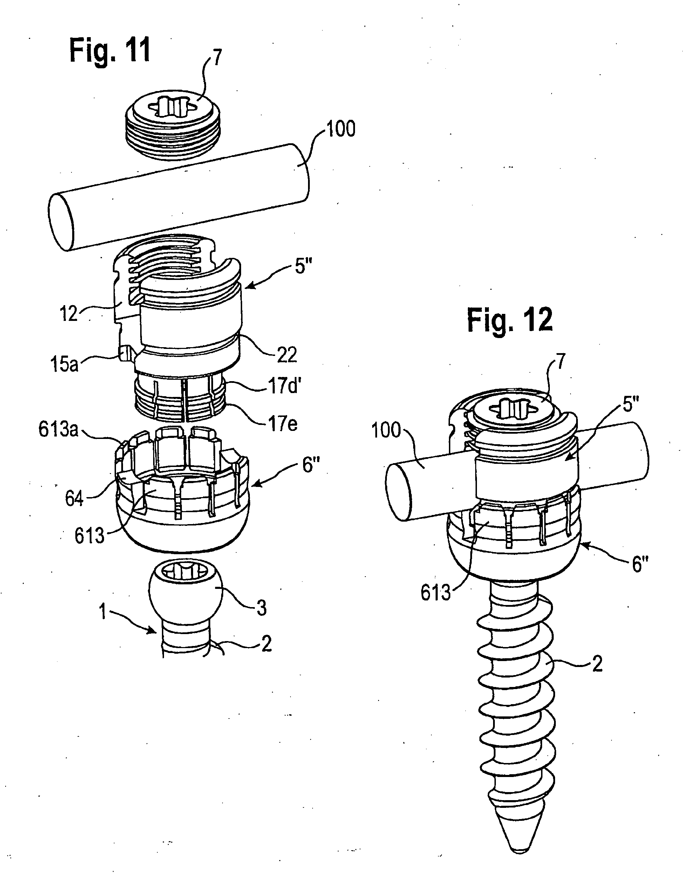

A third example of the bone anchoring device will now be described with reference to Figs. 11 to 15c). Parts and portions which are the same or similar to those of the first embodiment and the second example are designated with same reference numerals and the description thereof will not be repeated. The third example of the bone anchoring device differs from the first embodiment and the second example by the design of the pressure areas. In the third example pressure areas 21" are defined as circumferentially distinct projections which are located at an inner side of the locking ring 6".

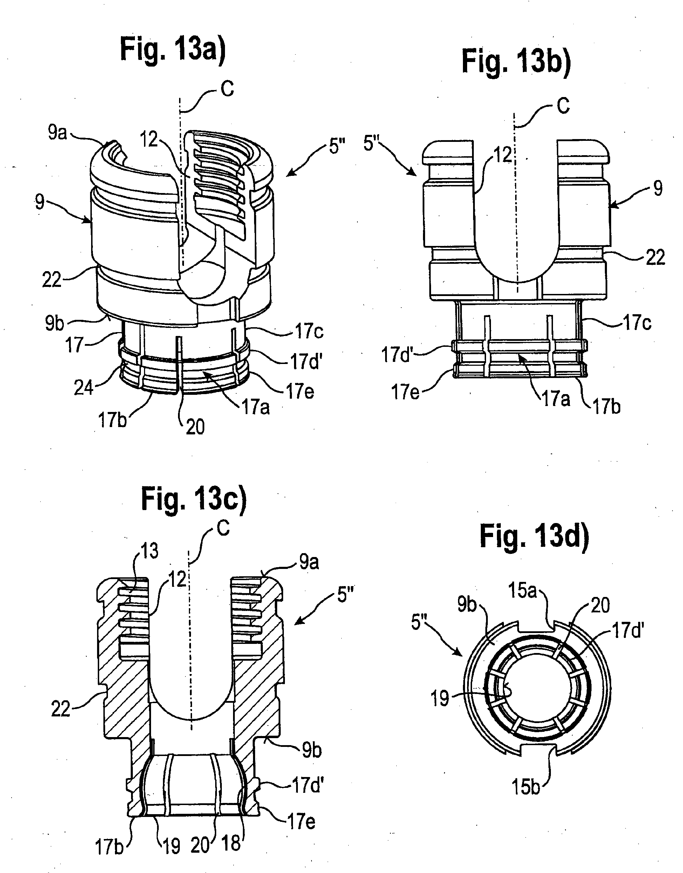

As can be seen in particular in Figs. 13a) to 13d) the receiving part body 5" has a head receiving portion 17 similar to that of the second example. The second cylindrical portion 17d' is provided at an axial position of the head receiving portion 17 which corresponds substantially to the largest outer diameter of the head 3. The slits 20 extend as in the first embodiment and the second example from the free end 17b to a distance from the second end 9b of rod receiving portion and on top of the second cylindrical portion 17d'.

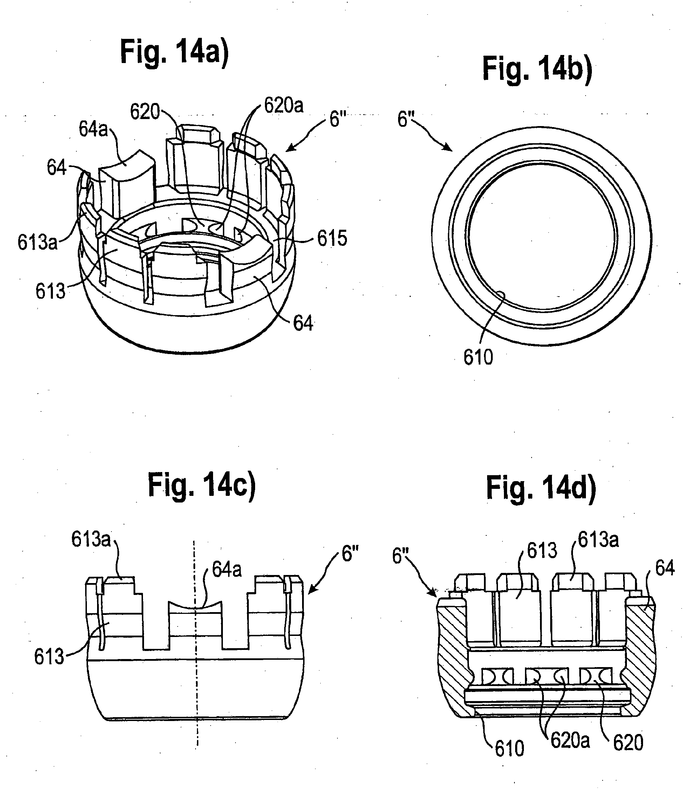

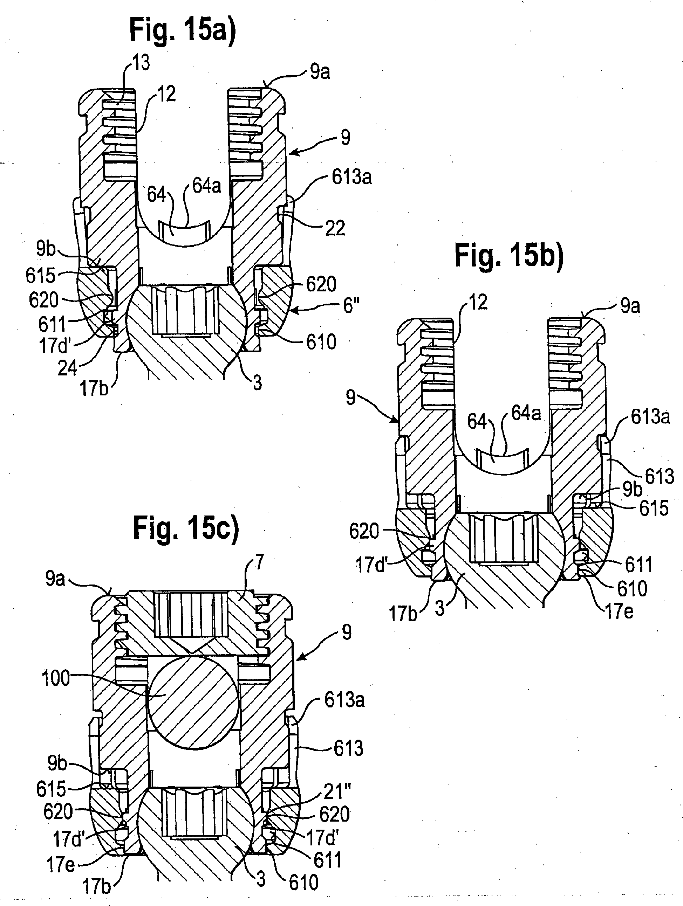

The locking ring 6" differs from the locking ring according to the second example in such a way that is has pressure areas in the form of circumferentially distinct projections 620 which are at an axial position corresponding to the position of the second cylindrical portion 17d' when the locking ring is in the third position as shown in Fig. 15c). The projections 620 project to the inside of the locking ring. Their number corresponds to the number of flexible wall segments 17a and the distance corresponds to the distance of the flexible wall sections 17a so that when the locking ring is mounted each projection 620 can engage one flexible wall section 17a. The projections 620 have a height in axial direction which is approximately the same as the height of the second cylindrical portion 17d'. Their width in a circumferential direction is smaller than the width of the flexible wall sections 17a thereby creating the distinct pressure area in each flexible wall section 17a. The projections can be generated by providing cut-outs of a projecting ring with inclined cutting surfaces 620a as shown in Fig. 14a) and Fig. 14d). However, other shapes are possible which are configured to generate a distinct pressure area with respect to the flexible wall sections 17a. Assembly and use of the bone anchoring device according to the third example is similar to that of the second example. As shown in Fig. 15a) in the first position, the inwardly projecting edge 610 of the locking ring 6" engages the groove 24 at the head receiving portion 17. The head can be introduced from the free end 17b.

In the second position, which is the pre-locking position, as shown in Fig. 15b) the engagement portions 613a engage the groove 22 at the rod receiving portion. In this position the projections 620 of the locking begin to press against the second cylindrical portion 17d' of the head receiving portion 17.

In the third position, as shown in Fig. 15c), the projections 620 are positioned substantially at the center of the second cylindrical portion 17d' and compress the flexible wall sections 17a to clamp the head 3. In addition, the inwardly projecting edge 610 engages the third cylindrical portion 17e. The combination of pressure of the projections 620 and the inwardly projecting edge 610 the head is firmly locked within the hollow internal section 18.

Single features of the embodiment described can be combined or exchanged with features of other examples. For example, it is possible to have the projections that form the distinct pressure areas on the flexible wall portions and on the locking ring alternately in a circumferential direction. The locking ring and the receiving part of the first embodiment can also be configured to have a pre-locking function and the locking ring and the receiving part of the second and third examplescan be designed without pre-locking function.

Further modifications of the embodiments shown are possible. For example, the head of the bone anchoring element can have another shape, such as, for example, a cylindrical shape, whereby a mono-axial bone screw is provided allowing rotation of the screw element with respect to the receiving part body around a single axis.

The projections forming the pressure areas can have any shape. Preferably, they have a rounded shape to facilitate movement of the locking ring.

The head receiving portion can have an inclined open end or can be otherwise asymmetric to allow a greater angulation of the head in one direction.

For the anchoring element, all kinds of anchoring elements can be used and combined with the receiving part. These anchoring elements are e.g. screws of different length, with different diameters, cannulated screws, screws with different thread forms, nails, hooks and anchoring elements wherein the head and the shaft are separate parts, etc.

The rod receiving portion may also have a different shape. For example, the recess can be configured to allow the rod to be introduced from the side instead of being introduced from the top. The recess can also be closed instead of U-shaped. Various kinds of locking devices, including two- or more-part locking device, outer nuts, outer caps, bayonet locking devices or others are possible.

## Claims

1. A receiving part for receiving a rod for coupling the rod to a bone anchoring element (1), the receiving part including
a receiving part body (5) with a top end (9a) and a bottom end (17b), and
a rod receiving portion (9) with a channel (12) for receiving the rod (100), and
a head receiving portion (17) for accommodating a head (3) of the bone anchoring element, the head receiving portion having an open end (19, 17b) for introducing the head (3); and
a locking ring (6) embracing the head receiving portion (17),
wherein the head receiving portion comprises a plurality of flexible wall portions (17a) and wherein the flexible wall portions (17a) and the locking ring (6) are configured to engage each other at circumferentially distinct pressure areas (21) within the flexible wall sections (17a), wherein
the distinct pressure areas are provided by tear drop-shaped projections (21), **characterized in that** the tear drop-shaped proj ections (21) are arranged at the centre of the flexible wall sections (17a) in an axial direction and in a circumferential direction.

2. The receiving part according to claim 1, wherein the flexible wall portions (17a) are separated by slits (20).

3. The receiving part according to claim 1 or 2, wherein the distinct pressure areas include circumferentially separated projections (21, 26) which are provided at the flexible wall sections (17a).

4. The receiving part of one of claims 1 to 3, wherein the tear drop-shaped projections are oriented such that the width and the height increases towards the open end (19, 17).

5. The receiving part of one of claims 1 to 4, wherein the locking ring (6) has a lower end (6b) oriented towards a free end of the head receiving portion (17) and wherein, adjacent to the lower end (6b), the locking ring (6) is substantially hollow cylindrically-shaped with an inner cylindrical surface (61), the diameter of which is slightly smaller than an outer diameter of the head receiving portion (17) in the region of the outermost area of the tear drop-shaped projections (21).

6. The receiving part of one of claims 1 to 5, wherein opposing surfaces of the head receiving portion (17) and the locking ring (6) other than at the distinct pressure areas are parallel.

7. The receiving part of one of claims 1 to 6, wherein the distinct pressure areas are arranged substantially at the centre of the flexible wall portion (17a), respectively.

8. The receiving part of one of claims 1 to 7, wherein the head receiving portion (17) includes a hollow internal section (18) for accommodating the head and wherein the distinct pressure areas (21) are located at a position of the largest inner diameter of the hollow internal section (18).

9. A bone anchoring device including a receiving part according to claims 1 to 8 and a bone anchoring element (1) having a shaft (2) for anchoring in the bone and a head (15).

## Patentansprüche

1. Ein Aufnahmeteil zum Aufnehmen eines Stabs zum Koppeln des Stabs an ein Knochenverankerungselement (1), wobei das Aufnahmeteil aufweist:
einen Aufnahmeteilkörper (5) mit einem oberen Ende (9a) und einem unteren Ende (17b), und
einen Stabaufnahmeabschnitt (9) mit einem Kanal (12) zum Aufnehmen des Stabs (100), und
einen Kopfaufnahmeabschnitt (17) zum Aufnehmen eines Kopfs (3) des Knochenverankerungselements, wobei der Kopfaufnahmeabschnitt ein offenes Ende (19, 17b) zum Einführen des Kopfs (3) besitzt; und
einen Verriegelungsring (6), welcher den Kopfaufnahmeabschnitt (17) umgreift,
wobei der Kopfaufnahmeabschnitt eine Vielzahl von flexiblen Wandabschnitten (17a) umfasst und wobei die flexiblen Wandabschnitte (17a) und der Verriegelungsring (6) eingerichtet sind, einander in umfänglich unterschiedlichen Druckbereichen (21) innerhalb der flexiblen Wandabschnitte (17a) anzugreifen, wobei
die unterschiedlichen Druckbereiche durch tropfenförmige Vorsprünge (21) bereitgestellt sind, **dadurch gekennzeichnet, dass** die tropfenförmigen Vorsprünge (21) in den flexiblen Wandabschnitten (17a) mittig in einer axialen Richtung und in einer Umfangsrichtung angeordnet sind.

2. Das Aufnahmeteil gemäß Anspruch 1, wobei die flexiblen Wandabschnitte (17a) voneinander durch Schlitze (20) getrennt sind.

3. Das Aufnahmeteil gemäß Anspruch 1 oder 2, wobei die verschiedenen Druckbereiche in Umfangsrichtung voneinander getrennte Vorsprünge (21, 26) aufweisen, welche an den flexiblen Wandabschnitten (17a) vorgesehen sind.

4. Das Aufnahmeteil gemäß einem der Ansprüche 1 bis 3, wobei die tropfenförmigen Vorsprünge solchermaßen orientiert sind, dass ihre Breite und ihre Höhe in Richtung auf das offene Ende (19, 17) zunimmt.

5. Das Aufnahmeteil gemäß einem der Ansprüche 1 bis 4, wobei der Verriegelungsring (6) ein unteres Ende (6b) besitzt, das in Richtung auf das freie Ende des Kopfaufnahmeabschnitts (17) orientiert ist, und wobei der Verriegelungsring (6) benachbart zu dem unteren Ende (6b) im Wesentlichen hohlzylinderförmig ist und eine innere zylindrische Oberfläche (61) aufweist, wobei der Durchmesser derselben geringfügig kleiner ist als ein Außendurchmesser des Kopfaufnahmeabschnitts (17) in dem am weitesten außen liegenden Bereich der tropfenförmigen Vorsprünge (21).

6. Das Aufnahmeteil gemäß einem der Ansprüche 1 bis 5, wobei einander gegenüberliegende Oberflächen des Kopfaufnahmeabschnitts (17) und des Verriegelungsrings (6), die nicht den unterschiedlichen Druckbereichen entsprechen, parallel zueinander sind.

7. Das Aufnahmeteil gemäß einem der Ansprüche 1 bis 6, wobei die unterschiedlichen Druckbereiche jeweils entsprechend im Wesentlichen in der Mitte der flexiblen Wandabschnitte (17a) angeordnet sind.

8. Das Aufnahmeteil gemäß einem der Ansprüche 1 bis 7, wobei der Kopfaufnahmeabschnitt (17) einen hohlen inneren Abschnitt (18) zum Aufnehmen des Kopfs aufweist, und wobei die unterschiedlichen Druckbereiche (21) an einer Position des größten Innendurchmessers des hohlen inneren Abschnitts (18) lokalisiert sind.

9. Eine Knochenverankerungsvorrichtung aufweisend ein Aufnahmeteil gemäß den Ansprüchen 1 bis 8 und ein Knochenverankerungselement (1) mit einem Schaft (2) zum Verankern in dem Knochen, sowie einen Kopf (15).

## Revendications

1. Partie de réception destinée à recevoir une tige pour le couplage de la tige à un élément d'ancrage osseux (1), la partie de réception comportant:
un corps de la partie de réception (5) avec une extrémité supérieure (9a) et une extrémité inférieure (17b), et
une partie de réception de tige (9) avec un canal (12) pour recevoir la tige (100), et
une partie de réception de tête (17) pour loger une tête (3) de l'élément d'ancrage osseux, la partie de réception de tête ayant une extrémité ouverte (19, 17b) pour introduire la tête (3) ; et
un anneau de verrouillage (6) entourant la partie de réception de tête (17),
dans laquelle la partie de réception de tête comprend une pluralité de parties de paroi flexibles (17a) et dans laquelle les parties de paroi flexibles (17a) et l'anneau de verrouillage (6) sont configurés pour s'engager ensemble au niveau de zones de pression circonférentiellement distinctes (21) à l'intérieur des sections de paroi flexibles (17a), dans laquelle
les zones de pression distinctes sont fournies par des saillies en forme de gouttes (21), **caractérisée en ce que** les saillies en forme de gouttes (21) sont agencées au centre des sections de paroi flexibles (17a) dans une direction axiale et dans une direction circonférentielle.

2. Partie de réception selon la revendication 1, dans laquelle les parties de paroi flexibles (17a) sont séparées par des fentes (20).

3. Partie de réception selon la revendication 1 ou 2, dans laquelle les zones de pression distinctes comportent des saillies circonférentiellement séparées (21, 26) qui sont pourvues au niveau des sections de paroi flexibles (17a).

4. Partie de réception de l'une des revendications 1 à 3, dans laquelle les saillies en forme de gouttes sont orientées de manière à ce que la largeur et la hauteur augmentent en direction de l'extrémité ouverte (19, 17).

5. Partie de réception de l'une des revendications 1 à 4, dans laquelle l'anneau de verrouillage (6) comporte une extrémité inférieure (6b) orientée en direction d'une extrémité libre de la partie de réception de tête (17) et dans laquelle, adjacent à l'extrémité inférieure (6b), l'élément de verrouillage (6) est de forme essentiellement cylindrique creuse avec une surface intérieure cylindrique (61), dont le diamètre est légèrement plus petit qu'un diamètre extérieur de la partie de réception de tête (17) dans la région de la zone ultrapériphérique des saillies en forme de gouttes (21).

6. Partie de réception de l'une des revendications 1 à 5, dans laquelle des surfaces opposées de la partie de réception de tête (17) et de l'anneau de verrouillage (6) autres que celles au niveau des zones de pression distinctes sont parallèles.

7. Partie de réception de l'une des revendications 1 à 6, dans laquelle les zones de pression distinctes sont agencées essentiellement au centre de la partie de paroi flexible (17a), respectivement.

8. Partie de réception de l'une des revendications 1 à 7, dans laquelle la partie de réception de tête (17) comporte une section interne creuse (18) pour loger la tête et dans laquelle les zones de pression distinctes (21) sont situées à une position du plus grand diamètre intérieur de la section interne creuse (18).

9. Dispositif d'ancrage osseux comprenant une partie de réception selon les revendications 1 à 8 et un élément d'ancrage osseux (1) ayant un arbre (2) pour l'ancrage dans l'os et une tête (15).
